(19) 

Europäisches
Patentamt

European
Patent Office

Office européen
des brevets

(11) **EP 4 483 815 A1**

(12) **EUROPEAN PATENT APPLICATION**

(43) Date of publication:
**01.01.2025 Bulletin 2025/01**

(21) Application number: **23306046.6**

(22) Date of filing: **28.06.2023**

(51) International Patent Classification (IPC):
***A61B 8/00*** (2006.01)　　　***G01S 7/52*** (2006.01)

(52) Cooperative Patent Classification (CPC):
**A61B 8/5207; A61B 8/461; A61B 8/465;
A61B 8/467; A61B 8/469; A61B 8/54;
G01S 7/52044; G01S 7/5205; G01S 7/52073**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventor: **ATTIA, Emmanuel
5656 Eindhoven (NL)**

(74) Representative: **Philips Intellectual Property &
Standards
High Tech Campus 52
5656 AG Eindhoven (NL)**

(54) **SYSTEM AND METHOD FOR TUNING ULTRASOUND IMAGING**

(57) A system and method are provided for tuning ultrasound imaging. The method includes displaying a first scan-converted ultrasound image, based on first scan lines of ultrasound imaging data, on a display (S411); receiving screen coordinates of a display point in the first scan-converted ultrasound image, where the display point has a corresponding depth (S412); transforming the screen coordinates of the display point to data coordinates of a corresponding data point in the first scan lines (S413); adjusting a parameter of the first scan lines at a scan depth of the data coordinates of the data point to provide second scan lines of the ultrasound imaging data, including the adjusted parameter (S414); performing scan conversion of the second scan lines to obtain a second scan-converted ultrasound image, where the second scan-converted ultrasound image is altered at the depth of the display point in response to the adjusted parameter (S415); and displaying the second scan-converted ultrasound image (S416).

FIG. 3

**Description**

FIELD OF THE INVENTION

The invention relates to a system and method for tuning ultrasound imaging.

BACKGROUND OF THE INVENTION

Ultrasound imaging has become indispensable for many medical imaging applications. An ultrasound imaging system typically includes an ultrasound transducer probe and a processing system. The ultrasound transducer probe may include an array of transducer elements configured to emit acoustic waves through a subject's body and to receive echo signals as the acoustic waves are reflected from the tissues, organs and other structures. The timing and strength of the echo signals generally correspond to the size, shape and mass of the structures in the subject's body, images of which are displayed to a user of the ultrasound imaging system, such as a sonographer or physician.

Ultrasound imaging data is acquired by the ultrasound transducer probes as scan lines, which are scan-converted in accordance with a post-processing algorithm to provide corresponding ultrasound images reconstructed for display. Scan conversion of ultrasound images is known in the art. US5528302A "Real-time ultrasound scan conversion" to Basoglu et al. describes a known scan conversion technique and is incorporated herein by reference in its entirety.

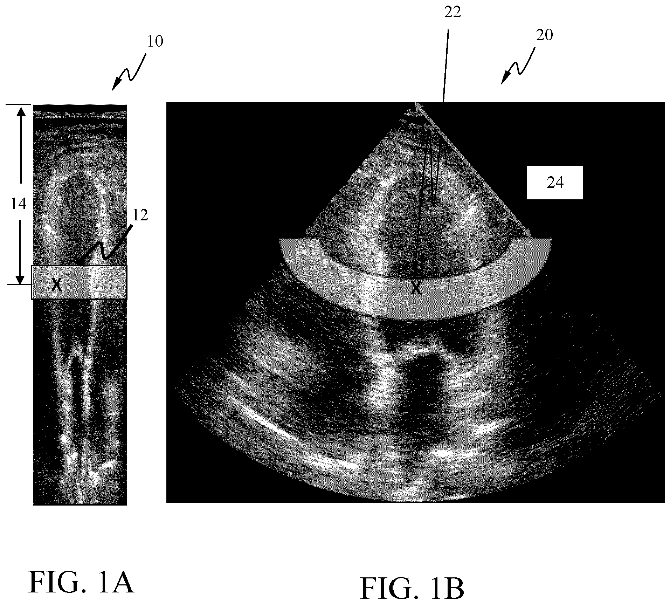

As an illustration, Fig. 1A shows ultrasound imaging data 10 as scan lines output by the ultrasound transducer probe before scan conversion, where for example the image data scan lines are arranged in a generally Cartesian coordinate system, where each data point 12 (X) is located at a particular depth 14 from the transducer probe face. Fig. 1B shows an exemplary scan-converted ultrasound image 20 corresponding to the ultrasound imaging data of Fig. 1A after scan conversion. FIG 1B for example shows the corresponding scan lines arranged in a polar data coordinate system that may more accurately correspond to the ultrasound image in the underlying anatomy. The data point 12 becomes thus scan-converted data point 22 (X) at radial depth 24. Generally, due to the anisotropic nature of the reconstructed ultrasound images after scan conversion, it would be useful to alter the strength of the post-processing algorithm by depth prior to the scan conversion. Pre-processing typically may be fined-tuned by depth using Time Gain Control (TGC) with a fixed number of sliders. However, because of the scan conversion, there is no trivial mapping between the geometry of the scan lines of ultrasound imaging data that are acquired, and the corresponding post-processed ultrasound image displayed on the display screen. Also, the relationship between the scan lines and the displayed ultrasound image depends on acquisition parameters and geometry of the ultrasound transducer probe.

Therefore, what is needed is a mechanism to adjust depth dependent parameters directly by interacting with the displayed ultrasound images and translating the adjustments to the scan lines to repeat processing. In ultrasound, image resolution is non-linearly dependent on depth particularly due to the curved geometry. Directly interacting with the displayed ultrasound images would enable the user to see results of the adjustments in real-time or near real-time to more quickly determine the accuracy of the settings, thereby improving accuracy of adjustments and quality of the final displayed ultrasound images.

SUMMARY OF THE INVENTION

The invention is defined by the independent claims. The dependent claims define advantageous embodiments.

According to an embodiment, a method is provided for tuning ultrasound imaging. The method includes displaying a first scan-converted ultrasound image on a display, where the first scan-converted ultrasound image is based on first scan lines of ultrasound imaging data; receiving screen coordinates of a display point in the first scan-converted ultrasound image, where the display point has a corresponding depth in the first scan-converted ultrasound image; transforming the screen coordinates of the display point in the first scan-converted ultrasound image to data coordinates of a corresponding data point in the first scan lines of the ultrasound imaging data; adjusting at least one parameter of the first scan lines at a scan depth of the data coordinates of the data point to provide second scan lines of the ultrasound imaging data, where the second scan lines include the adjusted at least one parameter, where the scan depth of the data coordinates corresponds to the depth of the display point in the first scan-converted ultrasound image; performing scan conversion of the second scan lines of the ultrasound imaging data to obtain a second scan-converted ultrasound image, where the second scan-converted ultrasound image is altered at the depth of the display point in response to the adjusted at least one parameter; and displaying the second scan-converted ultrasound image on the display.

According to another embodiment, a system is provided for tuning ultrasound imaging. The system includes a display, a user interface, a processing unit in communication with the display and the user interface, and a memory. The memory stores instructions that, when executed by the processing unit, cause the processing unit perform any of the herein disclosed methods.

According to yet another embodiment, a (non-transitory) computer readable medium stores instructions for tuning ultrasound imaging displayed on a display. When executed by a processing unit, the instructions cause the processing unit to perform any of the herein disclosed methods.

These and other aspects of the invention will be apparent from and elucidated with reference to the embodiments described hereinafter.

## BRIEF DESCRIPTION OF THE DRAWINGS

The representative embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.

Fig. 1A shows ultrasound imaging data as scan lines before scan conversion.
Fig. 1B shows an ultrasound image corresponding to the ultrasound imaging data of Fig. 1A after scan conversion.
Fig. 2 is a simplified block diagram of an ultrasound imaging system for tuning ultrasound imaging of a subject, according to an embodiment.
Fig. 3 is a simplified block diagram of memory in the ultrasound imaging system of Fig. 2, according to an embodiment.
Fig. 4 is a flow diagram of a method for tuning ultrasound imaging of a subject, according to an embodiment.
Fig. 5 is a schematic diagram of an integrated method and system for tuning ultrasound imaging of a subject, according to an embodiment.

## DETAILED DESCRIPTION OF EMBODIMENTS

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

As used in the specification and appended claims, and in addition to their ordinary meanings, the term "about" and "approximately" mean to with acceptable limits or degree. For example, "approximately 2 MHz" means one of ordinary skill in the art would consider the signal to be 2 MHz within reasonable measure. Also, as used in the specification and appended claims, in addition to its ordinary meaning, the term "substantially" means within acceptable limits or degree. For example, the term "substantially simultaneously" means one of ordinary skill in the art would consider occurrence at the same time.

Generally, according to various embodiments, a reconstructed ultrasound image is displayed on a display screen following scan conversion of corresponding ultrasound imaging data acquired by an ultrasound transducer probe. A user identifies a display point on the display screen having screen coordinates in the scan-converted ultrasound image. The screen coordinates of the display point are automatically transformed to data coordinates of a corresponding data point in a different coordinate system used before the scan conversion. The user may adjust a parameter of the data point at the data coordinates to effect a desired change in the displayed ultrasound image. Scan conversion is then performed on the ultrasound imaging data with the adjusted parameter in order to obtain a revised reconstructed ultrasound image. The parameter may be adjusted only by depth, although lateral adjustments may be possible using substantially the same technique, without departing from the scope of the present teachings.

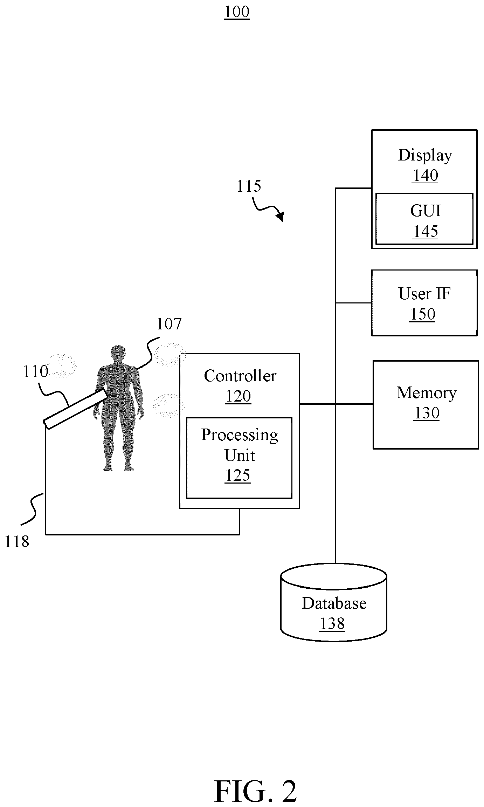

Fig. 2 is a simplified block diagram of an ultrasound imaging system for tuning post-processing ultrasound imaging of a subject, according to a representative embodiment.

Referring to Fig. 2, ultrasound imaging system 100 includes an ultrasound transducer probe 110 and a computer system 115 for controlling imaging of a region of interest (ROI) in or of a subject (patient) 107. The ultrasound transducer probe 110 may include a 2D matrix array of transducer elements, capable of scanning in two or three dimensions, for example, for emitting ultrasound waves into the body of the subject 107 and receiving echo signals in response. The transducer elements may include capacitive micromachined ultrasonic transducers (CMUTs) or piezoelectric transducers formed of materials such as lead zirconate titanate (PZT) or polyvinylidene difluoride (PVDF), for example, although other types of transducer material may be incorporated without departing from the scope of the present teachings. The transducer array is coupled to a microbeamformer in the ultrasound transducer probe 110, which controls transmission and reception of signals by the transducer elements.

The ultrasound transducer probe 110 is connected to a controller 120 in the computer system 115 via probe cable 118. The controller 120 includes processing unit 125, and is configured to control the ultrasound imaging process, as well as the process for tuning post-processing ultrasound imaging of the subject 107. The controller 120 includes known elements for performing ultrasound imaging, such as a transmit/receive (T/R) switch configured to switch between transmission and reception modes, e.g., under control of a user interface 150 (discussed below), and a main beamformer configured to provide final beamforming. One of the functions performed by the controller 120 is the direction in which beams are steered and focused. For example, beams may be steered straight ahead from (orthogonal to) the transducer array of the ultrasound transducer probe 110, or at different angles for a wider field of view. Generally, the transmitting of ultrasound signals and the receiving and processing of echo signals in response is known, and therefore additional detail in this regard is not included herein.

The computer system 115 receives ultrasound imaging data at the controller 120 from the ultrasound transducer probe 110 via the probe cable 118, and processes and stores the imaging data according to representative embodiments described herein. In addition to the controller 120, the computer system 115 includes memory 130, display 140, and the user interface 150. The display 140 has a display screen and may include a graphical user interface (GUI) 145 on the display screen. In alternative configurations, the computer system 115 may receive the ultrasound images from a database 138, which stores previously acquired ultrasound images of the subject 107.

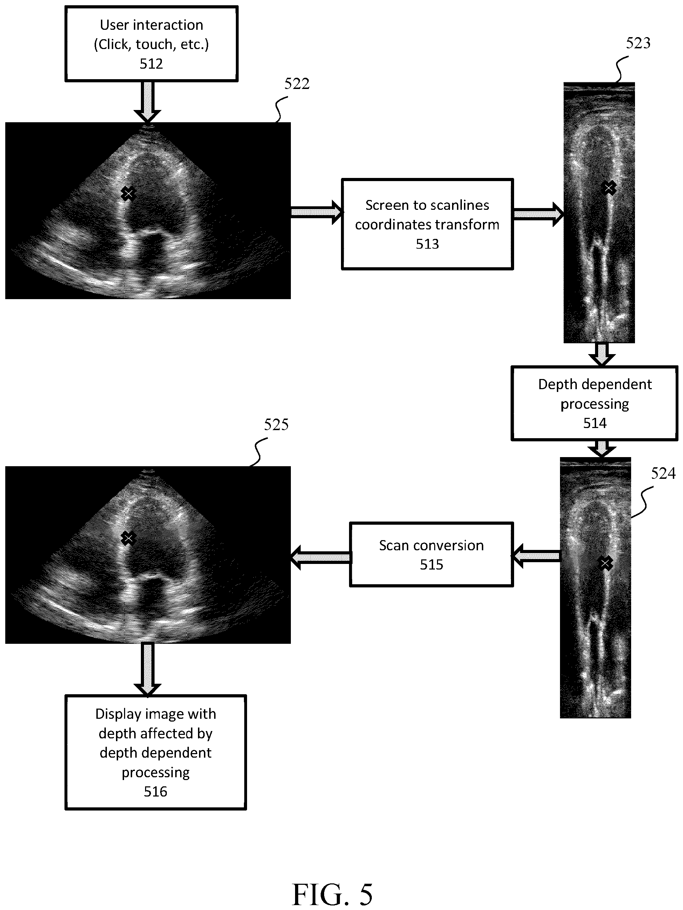

The controller 120 may receive the ultrasound imaging data as scan lines from the ultrasound transducer probe 110, although some standard preprocessing may have been performed before receiving the ultrasound imaging data. Alternatively, controller 120 may receive raw channel ultrasound data from the transducer probe 110 and construct scan lines itself. The scan lines may be provided in a data coordinate system of the ultrasound transducer probe 110. The controller 120 may additionally perform post-processing of the scan lines, and then scan-convert the post-processed scan lines to obtain corresponding scan-converted ultrasound images. The scan-conversion may be part of the post-processing in order to gain in precision, without departing from the scope of the present teachings. The scan-converted ultrasound images may be displayed on the display 140 in a screen coordinate system, as shown below in FIG 5, discussed below. The user may interact with the displayed scan-converted ultrasound images via the user interface 150 (e.g., implemented by the GUI 145) in order to identify one or more points or locations in the scan-converted ultrasound images, parameters of which the user would like to adjust, as discussed below. In addition, or alternatively, the ultrasound imaging data and/or the scan-converted ultrasound images may be stored in one or both of the memory 130 and the database 138.

The memory 130 stores instructions executable by the processing unit 125 of the controller 120. When executed, the instructions cause the processing unit 125 to perform various processes with regard to enabling tuning of scan-converted ultrasound images, as discussed below. The instructions further allow the user (e.g., sonographer, physician, or other clinician) to perform different steps of an ultrasound exam using the GUI 145 and/or the user interface 150, and to initialize the ultrasound transducer probe 110. In addition, the processing unit 125 may implement additional operations based on executing instructions, such as instructing or otherwise communicating with other elements of the computer system 115, including the memory 130, the display 140 and the user interface 150, to perform one or more of the processes described herein.

The processing unit 125 is representative of one or more processing devices, and is configured to execute software instructions stored in the memory 130 to perform functions as described in the various embodiments herein. The processing unit 125 may be implemented by a general purpose computer, a central processing unit (CPU), a graphics processing unit (GPU), a computer processor, a microprocessor, a microcontroller, a state machine, programmable logic device, field programmable gate arrays (FPGAs), application specific integrated circuits (ASICs), systems on a chip (SOC), or combinations thereof, using any combination of hardware, software, firmware, hard-wired logic circuits, or combinations thereof. Additionally, any processing unit or processor herein may include multiple processors, parallel processors, or both. Multiple processors may be included in, or coupled to, a single device or multiple devices.

The term "processor" as used herein encompasses an electronic component able to execute a program or machine executable instruction. References to "a processor" should be interpreted to include one processor or more than one processor or processing core, as in a multi-core processor. A processor may also refer to a collection of processors within a single computer system or distributed among multiple computer systems, such as in a cloud-based or other multi-site application. Modules have software instructions to carry out the various functions using one or multiple processors that may be within the same computing device or which may be distributed across multiple computing devices.

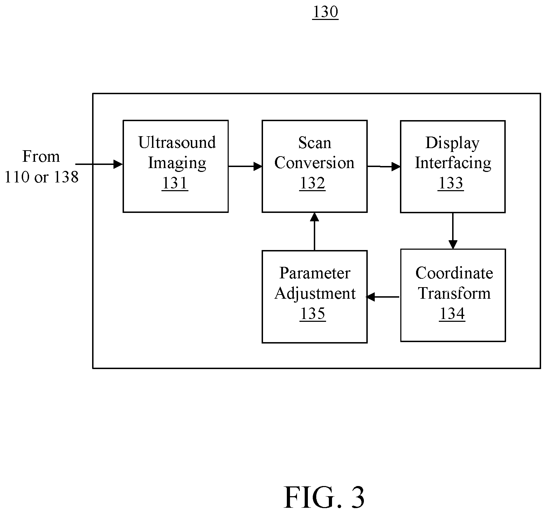

The memory 130 may include a main memory and/or a static memory, where such memories may communicate with each other and the processing unit 125 via one or more buses. The memory 130 stores instructions used to implement some or all aspects of methods and processes described herein. When executed, the instructions cause the processing unit 125 to implement one or more processes for tuning scan-converted ultrasound images described below with reference to Fig. 3, for example, as well as to control performance of the ultrasound imaging. The memory 130 may be implemented by any number, type and combination of random-access memory (RAM) and read-only memory (ROM), for example, and may store various types of information, such as software algorithms, which serves as instructions, which when executed by the processing unit 125 cause the processing unit 125 to perform various steps and methods according to the present teachings. Furthermore, updates to the methods and processes described herein may also be provided to the computer system 115 and stored in memory 130. The memory 130 may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium known in the art.

Each of the memory 130 and the database 138 is a (tangible) storage medium for storing data and executable software instructions, and may be non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. As discussed above, the memory 130 may store software instructions and/or computer readable code that enable performance of various functions, and the database 138 may store previously acquired ultrasound images of the subject 107. The memory 130 and/or the database 138 may be secure and/or encrypted, or unsecure and/or unencrypted.

"Memory" is an example of computer-readable storage media, and should be interpreted as possibly being multiple memories or databases. The memory or database for instance may be multiple memories or databases local to the computer, and/or distributed amongst multiple computer systems or computing devices, or disposed in the "cloud" according to known components and methods. A computer readable storage medium may be any medium that constitutes patentable subject matter under 35 U.S.C. §101 and excludes any medium that does not constitute patentable subject matter under 35 U.S.C. §101. Examples of such media include non-transitory media such as computer memory devices that store information in a format that is readable by a computer or data processing system. More specific examples of non-transitory media include computer disks and non-volatile memories.

The display 140 may be any compatible monitor for displaying at least ultrasound images, such as a computer monitor, a television, a liquid crystal display (LCD), a light emitting diode (LED) display, a flat panel display, a solid-state display, or a cathode ray tube (CRT) display, for example. The display 140 may also provide the GUI 145, discussed above, for displaying and receiving information to and from the user via the display screen. Although the display 140 is shown as a single display, it is understood that the ultrasound imaging system 100 may include multiple displays, for example, one of which is dedicated to displaying ultrasound images in real-time in response to manipulation of the ultrasound transducer probe 110, without departing from the scope of the present teachings.

The user interface 150 is configured to provide information and data output by the processing unit 125 and/or the memory 130 to the user and/or to receive information, instructions and data input by the user. That is, the user interface 150 enables the user to operate the ultrasound imaging system 100 as described herein, including entering imaging settings and ultrasound transducer probe settings, such as mode, frequency, depth and gain, for example. Notably, the user interface 150 enables the processing unit 125 to indicate the effects of the user's control or manipulation of the ultrasound transducer probe 110. The user interface 150 may include one or more of ports, disk drives, wireless antennas, or other types of receiver circuitry. The user interface 150 may further connect one or more interface devices, such as a mouse, a keyboard, a mouse, a trackball, a joystick, a microphone, a video camera, a touchpad, a touchscreen, voice or gesture recognition captured by a microphone or video camera, for example.

All or a portion of the user interface 150 may be implemented by the GUI 145 on the of the display 140, which may be a touch screen, for example. The user interface 150 may include graphics, such as buttons, fields, slides, and other visual markers displayed by the GUI 145 and operable by the user to initiate various commands for manipulating the displayed image, making measurements and calculations, and the like during the ultrasound examination. The push buttons may be displayed by the GUI 145 on the touch screen, for example.

Fig. 3 is a simplified block diagram of the memory 130, according to a representative embodiment. For purposes of illustration, the memory 130 is shown to include software modules, each of which includes sets of instructions, executable by the processing unit 125, corresponding to an associated capability of the ultrasound imaging system 100.

Referring to Fig. 3, ultrasound imaging module 131 is configured to receive ultrasound imaging data of the ROI in the subject 107, which may be acquired by the ultrasound transducer probe 110. The ultrasound imaging data may be received directly from the ultrasound transducer probe 110 in real-time or near real-time, e.g., during a contemporaneous imaging session of the subject 107. Alternatively, the ultrasound imaging data may be a previously acquired image that has been stored, e.g., in database 138, in which case the ultrasound imaging data may be retrieved from storage. The ultrasound imaging data may be received in the form of scan lines, an example of which is shown in Fig. 1A. The data points of the scan lines are represented by coordinates in a data coordinate system, which may be a two-dimensional (2D) Cartesian, polar or pseudo-polar coordinate system, for example.

Scan conversion module 132 is configured to perform known post-processing of the ultrasound imaging data, and to perform scan conversion of the scan lines following the post-processing to render a scan-converted ultrasound image that more closely corresponds to the actual geometry of the underlying imaged anatomy. Fig. 1B illustrates such a scan-converted image, for example. The post-processing may include various types of filtering, such as spatial filtering and speckle filtering, for example, as would be apparent to one skilled in the art. Generally, scan conversion maps the ultrasound imaging data from the data coordinate system to a screen coordinate system for display on the display 140. The data coordinate system may be a polar coordinate system and the screen coordinate system may be a Cartesian coordinate system, for example. Scan conversion may be performed using any compatible technique known to one skilled in the art.

Display interfacing module 133 is configured to display the scan-converted ultrasound image on the screen of the display 140, and to receive screen coordinates of a point in the displayed scan-converted ultrasound image ("display point") selected by the user via the user interface 150 and/or the GUI 145. The selected display point has a corresponding depth in the displayed scan-converted ultrasound image, where the depth is the distance from the transducer array in the ultrasound transducer probe 110 to the display point. The user may select the display point by simply identifying a location on the displayed scan-converted image by clicking on the location using a mouse, a trackball or a touchpad, or by touching the screen of the display 140 directly using a touchscreen, for example. Of course, other techniques enable selection of the location on the display 140 may be incorporated without departing from of the present teachings. Once the user has selected the location of the display point, the display interfacing module 133 identifies the screen coordinates in the screen coordinate system corresponding to the selected display point. Alternatively, the user may manually enter the specific coordinates of the selected display point.

Coordinate transform module 134 is configured to transform the screen coordinates of the selected display point in the displayed scan-converted ultrasound image to data coordinates of a corresponding data point in the scan lines of the ultrasound imaging data ("data point"). Transforming the screen coordinates of the selected display point to the data coordinates of the corresponding data point may be performed using a known transformation algorithm specific to the probe geometry of the ultrasound transducer probe 110. For example, the transformation algorithm to be used depends on the shape of the ultrasound transducer probe 110, and the values of the parameters of the transformation algorithm depend on the dimensions of the ultrasound transducer probe 110. For example, in 2D cardiovascular ultrasound imaging, transformation algorithm T inputs screen coordinates $(x, y)$ of the selected display point in the Cartesian screen coordinate system and outputs corresponding data coordinates $(\rho, \theta)$ of the corresponding data point in the polar data coordinate system, according to the following:

$$T: (x, y) =>$$

$$\rho\,(x, y) = \rho_{\text{scale}} * (\sqrt{(x^2 + y^2)} - \rho_0)$$

$$\theta\,(x, y) = \theta_{\text{scale}} * (\text{arctan2}(x, y) - \theta_0)$$

In the transformation algorithm T, $\rho$ is radial distance of the data point from a fixed origin, and $\theta$ is polar (lateral) angle. Also, $\rho_{\text{scale}}$ is radial resolution and $\theta_{\text{scale}}$ is azimuthal resolution, each of which depends on acquisition parameters of the scan lines. The values of $\rho_{\text{scale}}$ and $\theta_{\text{scale}}$ may be determined depending on the dimensions of the ultrasound probe 110, as mentioned above. Further, $\rho_0$ may be defined as the depth of the most proximal point of the acquisition, and $\theta_0$ may be defined as the minimum lateral angle.

Parameter adjustment module 135 is configured to receive instructions from the user via the user interface 150 and/or the GUI 145 to change value(s) of one or more post-processing parameters of the data point in the scan lines. For example, the GUI 145 may provide a grid or a drop-down list of parameters that are available for adjustment with regard to the displayed ultrasound image. The drop-down list may specify each potentially adjustable parameter, show the current parameter value, and provide a range of possible adjusted parameter values for selection. Alternatively, or in addition, the GUI 145 may provide an input field for the user to type in the one or more parameters to be adjusted, as well as the corresponding adjusted values. Of course, other forms of interfaces may be included, without departing from the scope of the present teachings. In any case, the adjustment may be indicated by a new parameter value to replace the current parameter value, or by an increased or decreased adjustment amount relative to the present parameter value. For example, the new parameter value may be presented as a percentage of the current parameter value, along with an indication of whether to increase or decrease the current parameter value by the percentage amount.

According to various embodiments, the parameters that may be adjusted are those dependent on scan depth, such as filtering strength, enhancement, grain, and contrast, for example, although this list is not exhaustive. Filtering strength refers to the degree to which noise or unwanted signals are removed (filtered) from the ultrasound imaging data. Generally, a higher filtering strength reduces noise, but may also remove some details from the ultrasound image. For example, there tends to be more noise in parts of the ultrasound image that is most dilated by the transformation, the filtering strength is increased for these parts, and since the increased filter strength provides less detail, these parts of the ultrasound image can be blurrier. Enhancement refers to brightness and darkness of the ultrasound image in order to enhance visibility of certain structures. Enhancement may be particularly useful in identifying subtle differences in tissue texture or identifying small structures, for example. Grain refers to the appearance of small dots or speckles in the ultrasound image, which may be caused by noise or other factors. Adjusting the grain parameter may be used to de-noise artifacts and to restore a more natural feeling of the ultrasound image. Contrast refers to the difference in brightness or darkness between different parts of the ultrasound image. Adjusting the contrast may be used to make certain structures more visible or distinguishable, especially in areas where the imaged tissue has similar echogenicity. It is understood that any additional parameters that are dependent on scan depth may be included as potentially adjustable parameters without departing from the scope of the present teachings.

The parameters of the data point at the scan depth in the scan lines may be adjusted as a function of the depth (distance) parameter in the polar data coordinate system. For example, for each of the data coordinates $(\rho, \theta)$ of the corresponding data point in the polar data coordinate system, the filtering may be modeled as follows, where "Parameters" is a set of constant parameters, such as filtering strength, enhancement, grain, and contrast, mentioned above:

$$\text{Output } (\rho, \theta) = f \text{ (Input, Parameters)}.$$

When the parameters are adjusted at a certain scan depth in the scan lines, they become a function of the depth coordinate $(\rho)$, as follows:

$$\text{Output } (\rho, \theta) = f \text{ (Input, Parameters, } \rho).$$

As mentioned above, the parameters of the ultrasound imaging data are dependent on scan depth. Accordingly, adjustments made to the value(s) of the one or more parameters in the scan lines may be applied to all of the data points at the same scan depth in the scan lines as the selected data point (corresponding to the selected display point). Alternatively, adjustments made to the value(s) of the one or more parameters of the scan lines may be applied only to the selected data point at the scan depth in the scan lines, leaving the value(s) of the same parameter(s) of the other data points at the scan depth unchanged. The user may select whether to apply the adjustments to all data points at the same depth or only the selected data point, for example, via the user interface 150 and/or the GUI 145.

Once the one or more parameters of the ultrasound imaging data have been adjusted, they are input to the scan conversion module 132. The post-processing of the ultrasound imaging data may then be repeated. The scan conversion module 132 then performs scan conversion of the scan lines with the adjusted parameters to provide a revised scan-converted ultrasound image. The revised scan-converted ultrasound image may be displayed on the display 140. If desired by the user, additional adjustments may be made to the one or more parameters, and/or to additional depth dependent parameters, by repeating the processes implemented by the display interfacing module 133, the coordinate transform module 134 and the parameter adjustment module 135.

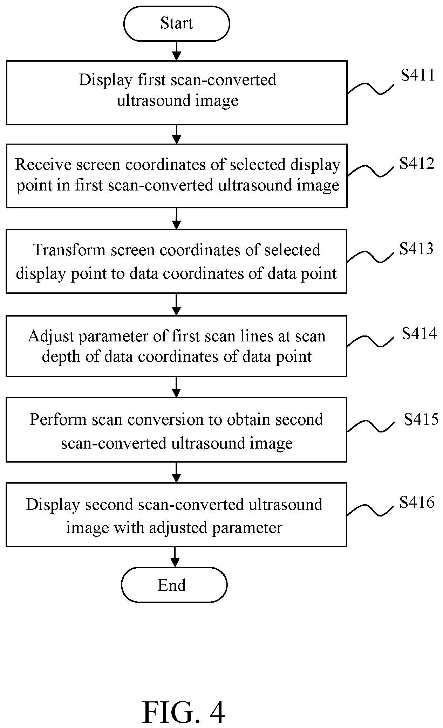

Fig. 4 is a flow diagram of a method for tuning post-processing ultrasound imaging of a subject, according to a representative embodiment. The method may be implemented by the ultrasound imaging system 100, discussed above, under control of the processing unit 125 executing instructions stored in the memory 130, for example.

Referring to Fig. 4, a first scan-converted ultrasound image is displayed on a display in block S411. The first scan-converted ultrasound image is rendered by post-processing first (initial) scan lines of ultrasound imaging data received from an ultrasound transducer probe, and scan converting the post-processed scan lines, as discussed above.

In block S412, screen coordinates of a selected display point in the displayed first scan-converted ultrasound image are received. The selected display point has a corresponding depth in the displayed first scan-converted ultrasound image. The selected display point may be identified by a user on the displayed first scan-converted ultrasound image. For example, the user may provide a user input by clicking on or touching a location in the displayed first scan-converted ultrasound image that corresponds to the selected display point, e.g., using a mouse, a trackball, a touch pad, or a touch screen. The selected display point has screen coordinates in a first coordinate system, such as a Cartesian coordinate system, for example, where the screen coordinates are automatically identified in response to the user selecting the selected display point.

In block S413, the screen coordinates of the selected display point are transformed to data coordinates of a data point in first scan lines of the ultrasound imaging data corresponding to the selected display point. The data coordinates are in a second coordinate system defined by the ultrasound transducer probe, such as a polar coordinate system, for example. The selected display point may be transformed to the corresponding data point by performing the Cartesian to polar transform discussed above with reference to coordinate transform module 134.

In block S414, at least one parameter of the first scan lines at a scan depth of the data coordinates of the data point is adjusted to provide second (adjusted) scan lines of the ultrasound imaging data. The adjustments to the at least one parameter may be received from the user input at the user interface, which may be implemented at least in part by a GUI, as discussed above. The second scan lines include the adjusted at least one parameter, which may be applied to the data point alone or to all data points at the same scan depth as the data point.

In block S415, scan conversion of the second scan lines of the ultrasound imaging data is performed to obtain a second scan-converted ultrasound image. The second scan-converted ultrasound image is altered at the depth of the display point in response to the adjusted at least one parameter of the data point prior to scan conversion, thereby providing an improved ultrasound image.

In block S416, the second scan-converted ultrasound image is displayed on the display. The second scan-converted ultrasound image may be displayed alone, or together with the first scan-converted ultrasound image, e.g., side by side or above/below one another in a split screen, to enable easy comparison by the user between the two ultrasound images for judging the effects of the parameter adjustment, as discussed below with reference to Fig. 5. When the first scan-converted ultrasound image is received from the ultrasound transducer probe and displayed on the display in real-time or near real-time, the second scan-converted ultrasound image may likewise be displayed on the display in real-time or near real-time. The process may be repeated by the user selecting another display point in the second scan-converted ultrasound image to be adjusted. The process then repeats by first returning to block S412, where screen coordinates of the additional selected display point are received, and so on.

Although methods, systems and components for implementing imaging protocols have been described with reference to several exemplary embodiments, it is understood that the words that have been used are words of description and illustration, rather than words of limitation. Changes may be made within the purview of the appended claims, as presently stated and as amended, without departing from the scope and spirit of the protocol implementation of the present teachings. The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

Fig. 5 is a schematic diagram of an integrated method and system for tuning post-processing ultrasound imaging of a subject, according to a representative embodiment.

Referring to Fig. 5, blocks 512 to 516 generally correspond to steps shown in blocks S412 to S416 of Fig. 4, respectively, while blocks 522 to 525 show images corresponding to the various steps. In block 512, user input is received at a user interface, such as a mouse click or a screen touch, for example. The user input identifies a selected display point (indicated by X) in a displayed first scan-converted ultrasound image 522. The selected display point has a corresponding depth in the displayed first scan-converted ultrasound image 522. The selected display point has screen coordinates in a first coordinate system, such as a Cartesian coordinate system, for example, which are automatically identified in response to the user input.

In block 513, the screen coordinates of the selected display point are transformed to data coordinates of a data point (indicated by X) in first scan lines 523 of ultrasound imaging data corresponding to the selected display point. The data coordinates in the first scan lines 523 are in a second coordinate system defined by the ultrasound transducer probe, such as a polar coordinate system, for example. The selected display point may be transformed to the corresponding data point by performing the Cartesian to polar transform discussed above with reference to coordinate transform module 134.

In block 514, depth dependent processing is performed to adjust at least one parameter of the first scan lines at a scan depth of the data coordinates of the data point to provide second (adjusted) scan lines 524 of the ultrasound imaging data. The adjustments to the at least one parameter may be entered by user input at the user interface, as discussed above. The depth dependent processing may include adjusting the at least one parameter of only the data point (indicated by X) or adjusting the at least one parameter of all data points at the same scan depth as the data point in the second scan lines 524. In block 515, scan conversion of the second scan lines 524 of the ultrasound imaging data is performed to obtain a second scan-converted ultrasound image 525. The second scan-converted ultrasound image 525 is altered at the depth of the display point (indicated by X) in response to the adjusted at least one parameter of the data point prior to scan conversion.

In block 516, the second scan-converted ultrasound image 525 is displayed on the display as the improved ultrasound image. The second scan-converted ultrasound image 525 may be displayed alone, or together with the first scan-converted ultrasound image 522, in response to user input. For example, the first scan-converted ultrasound image 522 and the second scan-converted ultrasound image 525 may be displayed in a split screen with the first scan-converted ultrasound image 522 displayed above the second scan-converted ultrasound image 525, as shown. Alternatively, the split screen may show the first scan-converted ultrasound image 522 and the second scan-converted ultrasound image 525 side by side. The split screen enables easy comparison by the user between the two ultrasound images for judging the effects of the parameter adjustment.

The Abstract is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

A computer program product may be software stored on a (non-transitory) medium. Alternatively, the computer program product may be software made available for download from some server, e.g. through the internet.

In the claims, any reference signs placed between parentheses shall not be construed as limiting the claim. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

**Claims**

1. A method of tuning ultrasound imaging, the method comprising:

   displaying (S411) a first scan-converted ultrasound image (20) on a display (140), wherein the first scan-converted ultrasound image is based on first scan lines of ultrasound imaging data (10);
   receiving (S412) screen coordinates of a display point (11) in the first scan-converted ultrasound image, wherein the display point has a corresponding depth in the first scan-converted ultrasound image;
   transforming (S413) the screen coordinates of the display point in the first scan-converted ultrasound image to data coordinates of a corresponding data point in the first scan lines of the ultrasound imaging data;
   adjusting (S414) at least one parameter of the first scan lines at a scan depth of the data coordinates of the data point to provide second scan lines of the ultrasound imaging data, wherein the second scan lines include the adjusted at least one parameter, wherein the scan depth of the data coordinates corresponds to the depth of the display point in the first scan-converted ultrasound image;
   performing (S415) scan conversion of the second scan lines of the ultrasound imaging data to obtain a second scan-converted ultrasound image, wherein the second scan-converted ultrasound image is altered at the depth of the display point in response to the adjusted at least one parameter; and
   displaying (S416) the second scan-converted ultrasound image on the display.

2. The method of claim 1, wherein the screen coordinates of the display point are received in response to a user input to display identifying the display point.

3. The method of claim 2, wherein the interaction by the user with the display includes at least one of a trackball operation, a trackpad touch, a mouse click or a touchscreen touch input.

4. The method of any of the preceding claims, wherein the at least one parameter of the first scan lines comprises at least one of: filtering strength, enhancement, grain or contrast.

5. The method of any of the preceding claims, wherein the at least one parameter is adjusted for all data coordinates at the scan depth in the first scan lines, including the data coordinates of the data point at the scan depth.

6. The method of any of claims 1 to 4, wherein the at least one parameter is adjusted for only the data coordinates of the data point at the scan depth in the first scan lines.

7. The method of any of the preceding claims, wherein transforming the screen coordinates of the display point in the first scan-converted ultrasound image to the data coordinates of the corresponding data point in the first scan lines of the ultrasound imaging data comprises:

   transforming the screen coordinates of the display point from a Cartesian screen coordinate system to the data coordinates of the corresponding data point in a polar data coordinate system using a transformation algorithm.

8. The method of claim 7, wherein the transformation algorithm comprises:

   $$T: (x, y) =>$$

   $$\rho\,(x, y) = \rho_{scale} * (\sqrt{(x^2 + y^2)} - \rho_0)$$

   $$\theta\,(x, y) = \theta_{scale} * (\arctan2(x, y) - \theta_0)$$

   wherein

   - $(x, y)$ are the screen coordinates of the display point in the Cartesian screen coordinate system,
   - $(\rho, \theta)$ are the data coordinates of the corresponding data point in the polar data coordinate system,
   - $\rho$ is radial distance of the corresponding data point from a fixed origin and
   - $\theta$ is polar angle.

9. The method of any of the preceding claims, wherein the at least one parameter of the first scan lines at the scan depth of the data coordinates of the data point is adjusted as a function of a depth coordinate in a polar screen coordinate system.

10. The method of any of the preceding claims, wherein the first scan-converted ultrasound image and the second scan-converted ultrasound image are displayed side by side.

11. A system for tuning ultrasound imaging, the system comprising:

    a display (140);
    a user interface (150); and
    a processing unit (125) configured to perform the method according to any of claims 1 to 10.

12. The system of claim 11, further comprising:
    an ultrasound transducer probe configured to acquire the ultrasound imaging data during an ultrasound examination of a subject, wherein the first scan-converted ultrasound image is displayed in real-time or near real-time during the ultrasound examination based on the first scan lines of the ultrasound imaging data acquired by the ultrasound transducer probe.

13. The system of any of claims 11 or 12, further comprising:
    a database storing the ultrasound imaging data previously acquired by an ultrasound transducer probe during an ultrasound examination of a subject, wherein the first scan-converted ultrasound image based on the first scan lines of the ultrasound imaging data retrieved from the database.

14. A computer program product (130) having instructions that, when executed by a processing unit (125), cause the processing unit to perform the method according to any of claims 1 to 10.

FIG. 1A

FIG. 1B

100

115

Display
140

GUI
145

User IF
150

Controller
120

Memory
130

Processing
Unit
125

107

110

118

Database
138

FIG. 2

130

From
110 or 138 → Ultrasound Imaging 131 → Scan Conversion 132 → Display Interfacing 133

Parameter Adjustment 135 → (to Scan Conversion 132)

Coordinate Transform 134 → Parameter Adjustment 135

FIG. 3

Start

Display first scan-converted
ultrasound image　　S411

Receive screen coordinates of selected display
point in first scan-converted ultrasound image　　S412

Transform screen coordinates of selected
display point to data coordinates of data point　　S413

Adjust parameter of first scan lines at scan
depth of data coordinates of data point　　S414

Perform scan conversion to obtain second
scan-converted ultrasound image　　S415

Display second scan-converted ultrasound
image with adjusted parameter　　S416

End

FIG. 4

User interaction
(Click, touch, etc.)
512

522

Screen to scanlines
coordinates transform
513

523

Depth dependent
processing
514

524

Scan conversion
515

525

Display image with
depth affected by
depth dependent
processing
516

FIG. 5

Europäisches
Patentamt

European
Patent Office

Office européen
des brevets

## EUROPEAN SEARCH REPORT

**Application Number**

**EP 23 30 6046**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2014/046185 A1 (MO RUOLI [CN] ET AL) 13 February 2014 (2014-02-13) * abstract * * paragraph [0024] – paragraph [0027] * * paragraph [0071] – paragraph [0084] * * figure 3 * | 1-14 | INV. A61B8/00 G01S7/52 |
| A | US 2016/228091 A1 (CHIANG ALICE M [US] ET AL) 11 August 2016 (2016-08-11) * abstract * * paragraph [0103] – paragraph [0110]; figures 3A, 3B, 4A * | 1-14 | |
| A | EP 3 907 700 A1 (KONINKLIJKE PHILIPS NV [NL]) 10 November 2021 (2021-11-10) * abstract * * paragraph [0041] – paragraph [0043] * | 1-14 | |

**TECHNICAL FIELDS SEARCHED (IPC)**

G01S
A61B

The present search report has been drawn up for all claims

| Place of search | Date of completion of the search | Examiner |
|---|---|---|
| Munich | 6 December 2023 | Montes, Pau |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 30 6046**

This annex lists the patent family members relating to the patent documents cited in the above-mentioned European search report.
The members are as contained in the European Patent Office EDP file on
The European Patent Office is in no way liable for these particulars which are merely given for the purpose of information.

**06-12-2023**

| Patent document cited in search report | | | Publication date | Patent family member(s) | | | Publication date |
|---|---|---|---|---|---|---|---|
| US 2014046185 | A1 | | 13-02-2014 | CN | 103455710 | A | 18-12-2013 |
| | | | | EP | 2702947 | A1 | 05-03-2014 |
| | | | | US | 2014046185 | A1 | 13-02-2014 |
| US 2016228091 | A1 | | 11-08-2016 | US | 2014121524 | A1 | 01-05-2014 |
| | | | | US | 2016228091 | A1 | 11-08-2016 |
| | | | | US | 2018168548 | A1 | 21-06-2018 |
| | | | | US | 2022125407 | A1 | 28-04-2022 |
| EP 3907700 | A1 | | 10-11-2021 | CN | 115516504 | A | 23-12-2022 |
| | | | | EP | 3907700 | A1 | 10-11-2021 |
| | | | | EP | 4147203 | A1 | 15-03-2023 |
| | | | | JP | 2023524543 | A | 12-06-2023 |
| | | | | US | 2023360224 | A1 | 09-11-2023 |
| | | | | WO | 2021224073 | A1 | 11-11-2021 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5528302 A